# EUROPEAN PATENT SPECIFICATION

(11) **EP 1 629 784 B1**
(45) Date of publication and mention of the grant of the patent: **13.01.2010**
(21) Application number: 05255238.7
(22) Date of filing: 25.08.2005
(51) Int. Cl.: A61B 17/22

(54) **Clot retrieval device**
Vorrichtung zur Entfernung von Gerinnseln
Dispositif de récupération de caillot

(30) Priority: 31.08.2004 US 605910 P; 04.08.2005 US 197662
(43) Date of publication of application: 01.03.2006
(73) Proprietor: Cordis Neurovascular, Inc., Miami Lakes, Florida 33014 (US)
(72) Inventor: West, Stephen, Pembroke Pines Florida 33028 (US)
(74) Representative: Gover, Richard Paul

(56) References cited:
- WO-A-98/02084
- WO-A-99/16382
- WO-A-2004/021922
- DE-U1- 8 626 340
- US-A- 5 549 626
- US-B1- 6 336 934
- PATENT ABSTRACTS OF JAPAN vol. 1999, no. 05, 31 May 1999 (1999-05-31) -& JP 11 047140 A (OLYMPUS OPTICAL CO LTD), 23 February 1999 (1999-02-23)

## Description

This invention generally relates to devices which are used to retrieve articles from vessels within a patient, and more particularly to devices which are employed to remove blood clots from blood vessels.

Each year many people suffer from strokes. The majority of strokes are caused by an occlusion, typically a blood clot, blocking blood flow to a portion of the brain- When a person is diagnosed with an occlusion-induced stroke, the time it takes to remove the occlusion is directly related to the amount of permanent damage suffered by the stroke victim. The longer it takes to remove the occlusion, the more permanent the damage can become.

There are typically two procedures that are used to unblock a blood vessel occluded by a blood clot. The first procedure requires administering a clot-busting drug which dissolves the blood clot to reestablish blood flow. One drawback to using such drugs is that it may take a couple of hours for the drug to dissolve the clot, if the clot dissolves at all.

The second type of procedure that has been used in the past utilizes an endovascular catheter in an approach which requires guiding a corkscrew shaped device, typically a corkscrew shaped wire, to the location of the blood clot. The blood clot is then snared by the corkscrew device, and removed from the patient. One of the problems associated with this procedure is that the corkscrew device does not enclose the clot. Thus, when the clot is being removed from the vessel, there is the possibility that pieces of the blood clot could break off, creating further complications.

WO-A-2004/021922 discusses an embolic filter. The filter comprises at least one pair of struts, one being a tension strut and the other a compression strut. Tethers are attached to an open edge of a filter membrane which is supported by the tension and compression struts when in an open configuration. The filter is deployed by changing the relative positions of three catheter tubes that are attached to the tethers, the compression struts and the tension struts, respectively.

Therefore, there remains a need that is recognized and addressed according to the present invention for an occlusion device which provides time efficient removal of blood clots, and reduces the risk of broken blood clot pieces from entering the blood stream.

The present invention generally relates to retrieval devices employed to remove articles, such as occlusions and more particularly blood clots, from vessels within a patient. Such retrieval device includes a retrieval unit comprising a pusher member having proximal and distal end portions. A retrieval basket is connected to the distal end portion of the pusher member. The retrieval basket is preferably a generally tubular member that exhibits an expanded state and a contracted state. The retrieval basket also includes a closeable opening located in the distal end portion of the retrieval basket. The closeable opening is adapted to receive an article, such as a blood clot, into the interior of the retrieval basket.

A control wire extends through the pusher member, through the retrieval basket and out of the opening located in the distal end portion of the retrieval basket. The control wire includes a distal end portion that extends distally of the distal end portion of the retrieval basket. The control wire is connected to the distal end portion of the retrieval basket by at least one tether. In this arrangement, the opening and closing of the retrieval basket and the transition between the expanded and contracted states of the retrieval basket can be achieved by movement of the control wire relative to the pusher member and the retrieval basket. The invention is characterised in that when the retrieval basket is in an expanded state with the opening fully opened, the at least one tether extends substantially perpendicular to a longitudinal axis of the control wire, such that relative axial movement of the control wire in a distal direction contracts the retrieval basket and closes the opening and relative axial movement of the control wire in a proximal direction closes the opening.

The distal end portion of the control wire is preferably comprised of a radiopaque material so that the control wire may also act as a guide wire, and thus eliminate the need for a guide catheter.

A suction source, for example a syringe, also may be associated with the pusher member. The suction source is utilized to create a suction force that facilitates movement of the clot into the retrieval basket. Typically, such movement is achieved by creating a pressure that is lower than that distal of the clot so as to "suction" the clot into the basket. In treating a blood clot, the distal end of a guiding catheter is placed at the site of blood clot using other devices and professional procedures generally known in the art. The pusher member is employed to guide the retrieval basket through the guiding catheter and out of the distal end portion of the guiding catheter. The retrieval basket may exit the guiding catheter by either advancing the pusher member or retracting the guide member, or both. The control wire is then used to adjust both the opening of the retrieval basket and the size of the retrieval basket, as desired by the operator. Once it is determined that the retrieval basket is arranged as desired, the operator activates the suction source. The clot is suctioned into the basket, and the control wire is then used to close down the opening of the basket. The pusher is then retracted and/or the guiding catheter is advanced to draw the retrieval basket containing the blood clot back into the guide catheter. The pusher member is then retracted out of the guiding catheter to remove the blood clot from the patient.

It is an object or aspect of the present invention to provide devices and methods that allow the removal of a blood clot from a patient in a time efficient manner.

It is also an object or aspect of the present invention to provide a device which reduces the risk of broken pieces of blood clots from entering the blood stream.

The retrieval basket may be a generally tubular element formed from a wall having apertures therethrough and may comprise a tubular braided element.

The suction source may be operably connected to the pusher member.

The present invention can be used in a method which does not form part of the claimed subject-matter, for retrieving an article from a vessel with a patient, comprising:
providing a retrieval unit comprising a retrieval basket capable of a transformation between an expanded state and a contracted state, a closeable opening located in a distal end portion of the retrieval basket, and a control member connected to the distal end portion of the retrieval basket;
placing the retrieval basket adjacent an article located in a vessel of the body;
drawing the article through the opening in the distal end portion of the retrieval basket into the retrieval basket;
closing the opening in the distal end portion of the retrieval basket by advancing or retracting the control wire; and
removing the retractable basket from the vessel.

The step of drawing may comprise suctioning so as to facilitate movement of the clot into the basket.

The method may include adjusting the opening in the distal end portion of the basket and the expanded and contracted states of the retrieval basket by advancing or retracting the control member.

The retrieval system of the present invention is designed for removing articles from vessels within a patient. However, for the sake of convenience, the description of the retrieval device herein will be described in terms of retrieving and removing a blood clot from the vasculature of a patient. It will be understood that the description herein does not limit the present invention to only such uses.

Embodiments of the invention will now be described by way of example with reference to the accompanying drawings, in which:
FIG. 1 is a longitudinal cross-sectional view of the retrieval system of a preferred embodiment of the present invention;
FIG. 2 is an enlarged perspective view of the distal end portion of the retrieval system of Fig. 1, shown with the retrieval basket in the expanded state;
FIG. 3 is an enlarged perspective view of the distal end portion of the retrieval system of Fig. 1, shown with the retrieval basket in the contracted state and with the opening closed down;
FIG. 4 is a partial perspective view of another preferred embodiment of the retrieval basket of the present invention;
FIGS. 5A and 5B are perspective side views of yet another preferred embodiment of the retrieval basket of the present invention; and
FIGS. 6A, 6B and 6C are schematic illustrations showing the retrieval device of Fig. 1 removing a blood clot from a blood vessel.

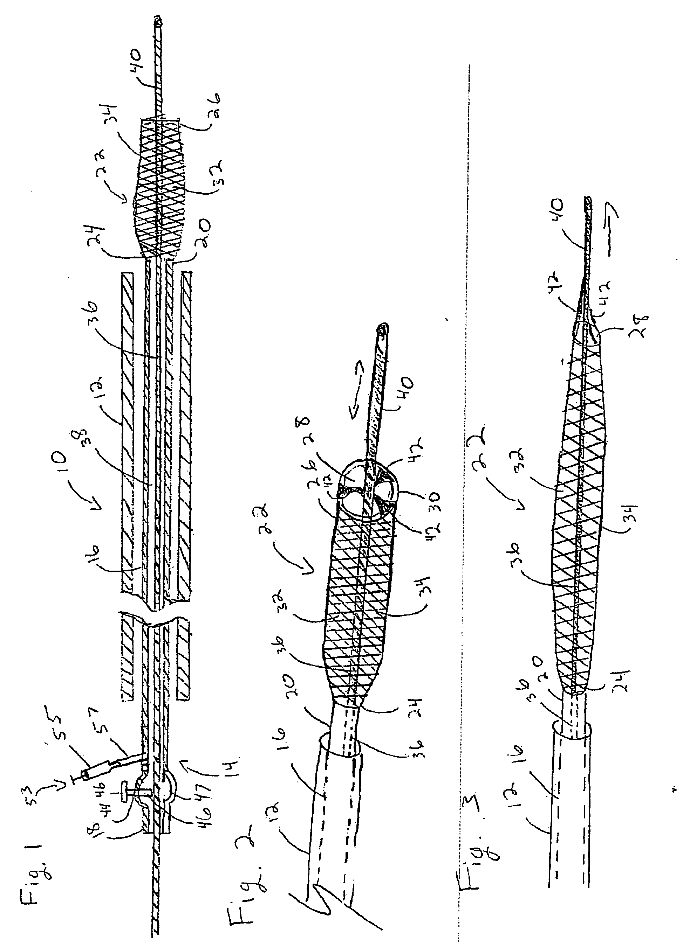

Fig. 1 illustrates a preferred embodiment of the retrieval system of the present invention. The retrieval system, generally designated at 10, may include an elongated flexible delivery tool or guiding catheter 12 which is inserted into the vasculature of a patient, and used to guide the retrieval unit, generally designated at 14, to the site of a blood clot in a blood vessel. This procedure is carried out in a manner generally known in the art.

The retrieval unit 14 includes an elongated flexible pusher member 16 which has a proximal end portion 18 and a distal end portion 20. A retrieval basket 22 is associated with the distal end portion 20 of the pusher member 16. Preferably, the retrieval basket 22 is a generally tubular element having a proximal end portion 24 and a distal end portion 26. The proximal end portion 24 of the retrieval basket 22 may be attached to the distal end portion 20 of the pusher member 16 by biocompatible adhesives, such as cyanoacrylate, UV curable adhesives or the like, or by any other suitable method of attachment. As illustrated in Fig. 2, an opening 28 is located in the distal end portion 26 of the retrieval basket 22. The opening 28 is adapted for receiving articles, such as blood clots, into the interior of the retrieval basket 22.

The structure of the retrieval basket 22 and the materials from which the retrieval basket may be made from may vary greatly. In one preferred embodiment, the retrieval basket may be formed to exhibit a stent-like structure. For example, as illustrated in Figs. 1-2, the generally tubular retrieval basket 22 may be formed from a wall 30 which has apertures 32 that extend therethrough to create a framework or mesh 34. Such a structure may be constructed by laser-cutting a hypo-tube using devices and procedures that are generally known in the art. The apertures 32 assist in transitioning the retrieval basket from an expanded state (as can be seen in Fig. 2) to a contracted state (as can be seen in Fig. 3), which will be described in more detail below. In this embodiment the retrieval basket 22 is preferably made of a metal, such as stainless steel, nitinol or the like. However, it is also contemplated that this structure may also be constructed using different methods and different materials, such as flexible and elastic polymers.

It is also contemplated that the retrieval basket 22 could be constructed of a braided element. The braided element could be constructed from a multi-stranded braid wherein the braids may comprise a metal, a fabric, a polymer or any combination of metal, fabric or polymer braids. Further, in either embodiment, the retrieval basket may be covered or encased, wholly or partially, by a flexible membrane (not shown). The flexible membrane preferably is comprised of an elastomeric material, such as medical urethane (such as that sold under the trade name Tecoflex or Tecothane) or Teflon.

As illustrated in Figs. 1-3, the retrieval unit 14 also includes a core or control wire 36. The control wire 36 preferably extends through a lumen 38 located in the pusher member 16. The control wire 36 also extends through the interior of the retrieval basket 22 and out of the opening 28. The control wire has a distal end portion 40 that extends beyond distal end portion 26 of the retrieval basket 22. The control wire 36 is preferably made of a metal, such as stainless steel. The distal end portion 40 of the control wire 36 may be configured into any shape desired and according to needs of a particular use, and the distal portion may also be made of a radiopaque material, such as platinum or tungsten. Thus, it will be understood that the distal end portion 40 can be made of a different material than the rest of the control wire 36. Alternatively, the radiopaque characteristic can be imparted by one or more bands or rings (not shown) of radiopaque material. The radiopaque distal end portion 40 allows the control wire 36 to act as a guide wire and aids in accurate placement of the device within a vessel of a patient. Thus, when time is of the essence and when situations and conditions otherwise permit, the use of the guide catheter may be eliminated, and the radiopaque distal end 40 of control wire 36 may be relied upon to guide the retrieval unit 14 to the site of the blood clot.

As illustrated in Figs. 2 and 3, the distal end portion 26 of the retrieval basket 22 adjacent the opening 28 is connected to the control wire 36 by one or more tethers 42. As illustrated, the retrieval basket 22 is connected to the core wire 36 by three tethers; however, it should be understood that any suitable number of tethers 42 could be used. The tethers 42 may be made of a polymer, such as nylon fiber, or a metal, such as stainless steel web or thread or any other suitable material. The tethers 42 may be attached to the retrieval basket 22 and to the control wire 36 by any suitable method, including but not limited to biocompatible adhesives, solder or welding.

With the above arrangement, the control wire 36 may be used to control the opening and the closing of the opening 28 at the distal end portion 26 of the retrieval basket 22, and also may be used to control the expanding and contracting of the retrieval basket. Fig. 2 illustrates the retrieval basket 22 in its expanded position with the opening fully opened. In a preferred arrangement, the basket is biassed to achieve its open condition when not otherwise constrained such as by a tether. Thus, the basket will be closed by pulling action of the tether(s). Otherwise, the tether(s) could exhibit adequate rigidity to open the basket opening when in a location and position as illustrated in Fig. 2.

As illustrated by the arrows in Fig. 2, the guide wire may be advanced or retracted axially in the proximal or distal direction. As illustrated in Fig. 3, when the control wire 36 is advanced and/or the distal basket 22 is moved in a retrograde manner, the retrieval basket 22 contracts, and the opening 28 closes down and becomes smaller. It should also be noted that, if the control wire 36 is retracted and/or the basket is advanced distally from their respective positions shown in Fig. 2, the tether(s) 42 will move into the interior of the retrieval basket 22, the effect of which will be to close down the opening 28. Thus, the control wire may be used to adjust the size of the opening and the degree of expansion or contraction of the retrieval basket as desired.

The pusher member 16 may also include a lock 44 for locking the control wire 36 in the desired position. The lock 44 is preferably located at the proximal end portion 18 of the pusher member 16, and is generally illustrated as a nut-lock that may be rotated by handle 46 to lock and unlock the control wire 36. In the locked position, the end 47 of the lock presses the control wire 36 against the inner wall 48 of the pusher member 16, locking the wire in position. When it is desired to advance or retract the control wire 36, the handle 46 of the lock is turned to move the distal end 47, releasing the control wire 36 to allow movement of the same.

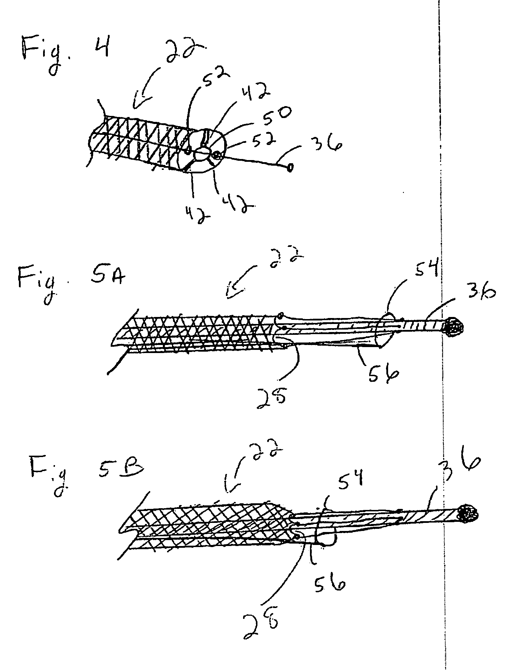

Figs. 4 and 5A and 5B illustrate alternative embodiments of the retrieval basket and control wire. In Fig. 4, the tethers 42 are attached to the control wire 36 by a slip ring 50 which has been placed over the control wire. The slip ring 50 is positioned over the control wire 36 between a pair of retaining members 52. The retaining members 52 retain the slip ring 50 over the designated portion of the control wire 36 between the retainer members 52 as the control wire is advanced and retracted.

In Figs. 5A and 5B, a hoop-like member such as a D-ring 54 is shown placed over the control wire 36 and the tethers 42. A pull wire 56 is connected to the member or D-ring 54 so that when the operator pulls the pull wire 56, the opening 28 at the distal end portion of the retrieval basket 22 will close down. That is, when the operator pulls the pull wire 56, the member or D-ring 54 will move proximally to a position such as shown in Fig. 5B, causing the tethers 42 to move closer to the control wire 36, resulting in closing down of the opening 28.

As illustrated in Fig. 1, the retrieval unit 14 may also include a suction source 53 associated with the proximal end 18 of the pusher member 16. The suction source 53 is generally illustrated as a syringe 55 connected to the pusher member 16 by suction port 57. The suction source is in fluid-passing communication with the lumen 38 of the pusher member 18. When the suction source 53 is activated, it creates a suction force that suctions the blood clot into the retrieval basket as generally described herein.

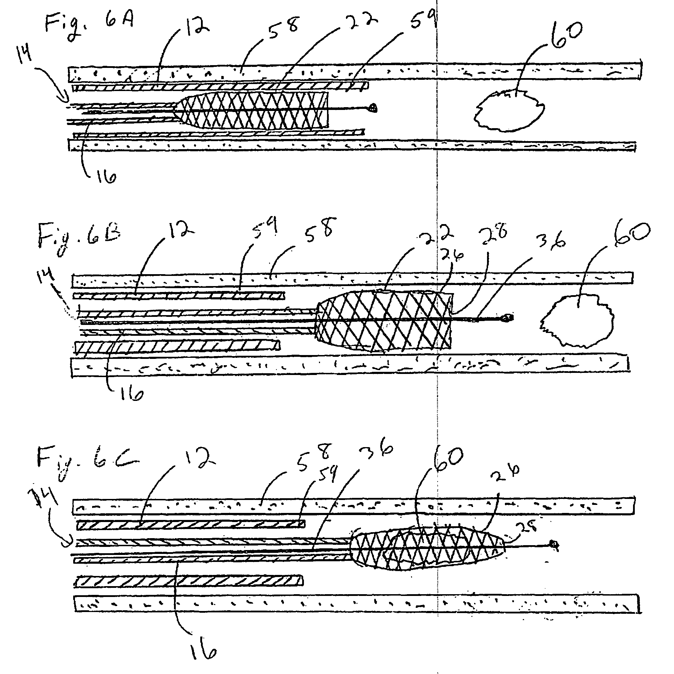

Figs. 6A, 6B and 6C schematically illustrate one embodiment of the retrieval device of the present invention retrieving a blood clot from a blood vessel within a patient. Consistent with other statements herein, this description of suitable uses is in no way meant to limit the present invention to only such uses.

As illustrated in Fig. 6A, in operation, the delivery catheter 12 is inserted into the vasculature of a patient and positioned at a preselected location within a vessel 58, typically in conjunction with other devices and professional procedures as generally known in the art. The retrieval unit 14 is inserted into the delivery catheter 12, and the pusher member 16 guides the retrieval basket 22 to the distal end 59 of the delivery catheter 12. As illustrated in Fig. 6B, once the retrieval basket 22 has reached the distal end 59 of the delivery catheter 12, the pusher member 16 is advanced and/or the delivery catheter 12 is moved in retrograde fashion until the retrieval basket 22 has exited the distal end portion 59 of the delivery catheter.

The pusher member 16 is then used to position the retrieval basket 22 into the desired position. The operator may then use the control wire 36 to adjust the retrieval basket 22, in a manner as described above. Once the retrieval basket 22 is in the desired position and is in the desired arrangement, the operator may lock the control wire 36 in place with lock 44, if desired. The suction source 53 is then activated to suction the blood clot 60 through the opening 28 and into the retrieval basket 22.

As illustrated in Fig. 6C, after the blood clot 60 has been received into the retrieval basket 22, the control wire 36 may be unlocked, and either advanced or retracted with respect to the pusher member 16 and/or the pusher member 16 may be advanced or retracted with respect to the control wire 36 to close down the opening 28 of the distal end portion 26 of the retrieval basket 22. The pusher member 16 may then be used to retract the retrieval basket 22 containing the blood clot 60 from the patient back into the delivery catheter 12 and out of the patient. It should be understood that, if the retrieval basket 22 and the blood clot 60 are too large to retract into the delivery catheter 12, the entire retrieval system 10 may be withdrawn from the patient.

It will be understood that the present invention may be used in conjunction with other medical procedures and devices, and also in conjunction with drug therapies. It will be understood that the embodiments of the present invention which have been described are illustrative of the present invention as defined in claim 1.

## Claims

1. A retrieval system for retrieving articles from vessels within a patient, comprising:
a retrieval unit (14) including a pusher member (16) having a proximal end portion (18), a distal end portion (20) and a lumen extending from the proximal end portion of the pusher member to the distal end portion of the pusher member;
a retrieval basket (22) capable of transforming between an expanded state and a contracted state, said retrieval basket including a proximal end portion (24) and a distal end portion (26), said proximal end portion (24) of the retrieval basket being connected to the distal end portion (20) of the pusher member (16);
a closeable opening (28) located in the distal end portion (26) of the retrieval basket (22), said opening (28) being sized and shaped for receiving an article located within a vessel into the said retrieval basket (22); and
a control wire (36) connected to the distal end portion (26) of the retrieval basket by at least one tether (42), wherein the control wire (36) extends through the lumen of the pusher member (16) and the opening in the distal end portion (26) of the retrieval basket (22) and includes a distal portion (40) that extends distally of the distal end portion (26) of the retrieval basket (22), and said control wire (36) controls the transformation of the retrieval basket (22) between the expanded state and the contracted state and opening and closing of the opening (28) in the distal end portion (26) of the retrieval basket (22);
**characterised in that** when the retrieval basket (22) is in an expanded state with the opening (28) fully opened, the at least one tether (42) extends substantially perpendicular to a longitudinal axis of the control wire (36), such that axial movement of the control wire (36) in a distal direction relative to the pusher member (16) contracts the retrieval basket (22) and closes the opening (28) and axial movement of the control wire (36) in a proximal direction relative to the pusher member (16) closes the opening (28).

2. The retrieval system of claim 1 wherein at least the distal portion (40) of the control wire comprises a radiopaque material.

3. The retrieval system of claim 1 further including a suction source (53) for suctioning an article within the vessel into the retrieval basket (22).

4. The retrieval system of claim 3 wherein the suction source (53) comprises a syringe (55) associated with the pusher member (16).

5. The retrieval system of claim 1 wherein the retrieval basket (22) is formed from a wall having apertures extending through the wall.

6. The retrieval system of claim 1 wherein the retrieval basket (22) comprises a tubular braided element.

7. The retrieval system of claim 1 wherein the retrieval basket (22) is constructed of a material including a metal.

8. The retrieval system of claim 1 wherein said tether (42) is connected to the control wire via a slip ring (50) positioned over the control wire (36) between a pair of retaining members (52).

9. The retrieval system of claim 1 further including a wire lock (44) associated with the pusher member (16), and said wire lock (44) locks the control wire (36) in place relative to the pusher member (16).

## Patentansprüche

1. Holsystem zum Herausholen von Gegenständen aus Gefäßen innerhalb eines Patienten, welches Folgendes umfasst:
Eine Holeinheit (14), welche ein Schubelement (16) aufweist, welches einen proximalen Endbereich (18), einen distalen Endbereich (20) und ein Lumen besitzt, welches sich von dem proximalen Endbereich des Schubelements zu dem distalen Endbereich des Schubelements erstreckt;
einen Holkorb (22), welcher geeignet ist, sich zwischen einem expandierten Zustand und einem zusammengezogenen Zustand umzuwandeln, wobei der Holkorb einen proximalen Endbereich (24) und einen distalen Endbereich (26) aufweist, wobei der proximale Endbereich (24) des Holkorbes mit dem distalen Endbereich (20) des Schubelements (16) verbunden ist;
eine verschließbare Öffnung (28), welche sich in dem distalen Endbereich (26) des Holkorbes (22) befindet, wobei die Öffnung (28) bemessen und geformt ist, um einen Gegenstand, welcher sich innerhalb eines Gefäßes befindet, in den Holkorb (22) aufzunehmen; und
einen Steuerungsdraht (36), welcher mit dem distalen Endbereich (26) des Holkorbes durch zumindest ein Anbindungselement (42) verbunden ist, wobei sich der Steuerungsdraht (36) durch das Lumen des Schubelements (16) und die Öffnung in dem distalen Endbereich (26) des Holkorbs (22) erstreckt und einen distalen Bereich (40) aufweist, welcher sich distal zu dem distalen Endbereich (26) des Holkorbes (22) erstreckt, und wobei der Steuerungsdraht (36) die Umwandlung des Holkorbs (22) zwischen dem expandierten Zustand und dem zusammengezogenen Zustand und ein Öffnen und Schließen der Öffnung (28) in dem distalen Endbereich (26) des Holkorbs (22) steuert;
**dadurch gekennzeichnet, dass** sich das zumindest eine Anbindungselement (42) im Wesentlichen senkrecht zu einer Längsachse des Steuenungsdrahtes (36) erstreckt, wenn sich der Holkorb (22) in, einem expandierten Zustand mit vollständig geöffneter Öffnung (28) befindet, sodass eine axiale Bewegung des Steuerungsdrahtes (36) in eine distale Richtung relativ zu dem Schubelement (16) den Holkorb (22) zusammenzieht und die Öffnung (28) schließt, und wobei eine axiale Bewegung des Steuerungsdrahtes (36) in eine proximale Richtung relativ zu dem Schubelement (16) die Öffnung (28) schließt.

2. Holsystem nach Anspruch 1, wobei zumindest der distale Bereich (40) des Steuerungsdrahtes ein röntgenstrahlenundurchlässiges Material umfasst.

3. Holsystem nach Anspruch 1, welches weiterhin eine Saugquelle (53) zum Ansaugen eines Gegenstands innerhalb des Gefäßes in den Holkorb (22) aufweist.

4. Holsystem nach Anspruch 3, wobei die Saugquelle (53) eine Spritze (55) umfasst, welche mit dem Schubelement (16) in Verbindung steht.

5. Holsystem nach Anspruch 1, wobei der Holkorb (22) aus einer Wand geformt ist, welche Löcher aufweist, die sich durch die Wand erstrecken.

6. Holsystem nach Anspruch 1, wobei der Holkorb (22) ein röhrenförmiges geflochtenes Element umfasst.

7. Holsystem nach Anspruch 1, wobei der Holkorb (22) aus einem Material aufgebaut ist, welches ein Metall aufweist.

8. Holsystem nach Anspruch 1, wobei das Anbindungselement (42) mit dem Steuerungsdraht mittels eines Schleifrings (50) verbunden ist, welcher über dem Steuerungsdraht (36) zwischen einem Paar von Rückhalteelementen (52) positioniert ist.

9. Holsystem nach Anspruch 1, welches weiterhin eine Drahtarretierung (44) aufweist, die mit dem Schubelement (16) in Verbindung steht, und wobei die Drahtarretierung (44) den Steuerungsdraht (36) relativ zu dem Schubelement (16) arretiert.

## Revendications

1. Système de récupération permettant de récupérer des articles provenant de vaisseaux dans un patient, comprenant :
➢ une unité de récupération (14) comprenant un élément pousseur (16) ayant une partie d'extrémité proximale (18), une partie d'extrémité distale (20) et une lumière s'étendant depuis la partie d'extrémité proximale de l'élément pousseur jusqu'à la partie d'extrémité distale dudit élément pousseur,
➢ un panier de récupération (22) capable de se transformer entre un état expansé et un état contracté, ledit panier de récupération comprenant une partie d'extrémité proximale (24) et une partie d'extrémité distale (26), ladite partie d'extrémité proximale (24) du panier de récupération étant reliée à la partie d'extrémité distale (20) de l'élément pousseur (16) ;
➢ une ouverture refermable (28) située dans la partie d'extrémité distale (26) du panier de récupération (22), ladite ouverture (28) étant dimensionnée et façonnée pour recevoir un article situé dans un vaisseau dans ledit panier de récupération (22) ; et
➢ un câble de commande (36) connecté à la partie d'extrémité distale (26) du panier de récupération par au moins une attache (42), dans lequel le câble de commande (36) s'étend à travers la lumière de l'élément pousseur (16) et l'ouverture dans la partie d'extrémité distale (26) du panier de récupération (22) et comprend une partie distale (40) qui s'étend de manière distale, depuis la partie d'extrémité distale (26) du panier de récupération (22) et ledit câble de commande (36) commande la transformation du panier de récupération (22) entre l'état expansé et l'état contracté et l'ouverture et la fermeture de l'ouverture (28) dans la partie d'extrémité distale (26) du panier de récupération (22) ;
**caractérisé en ce que**, lorsque le panier de récupération (22) est dans un état expansé avec l'ouverture (28) totalement ouverte, ladite au moins une attache (42) s'étend sensiblement perpendiculairement à un axe longitudinal du câble de commande (36), de sorte que le mouvement axial du câble de commande (36) dans une direction distale relativement à l'élément pousseur (16) contracte le panier de récupération (22) et ferme l'ouverture (28) et que le mouvement axial du câble de commande (36) dans une direction proximale relativement à l'élément pousseur (16) ferme l'ouverture (28).

2. Système de récupération selon la revendication 1, dans lequel au moins la partie distale (40) du câble de commande comprend un matériau radio-opaque.

3. Système de récupération selon la revendication 1, comprenant en outre une source d'aspiration (53) pour aspirer un article du vaisseau dans le panier de récupération (22).

4. Système de récupération selon la revendication 3, dans lequel la source d'aspiration (53) comprend une seringue (55) associée à l'élément pousseur (16).

5. Système de récupération selon la revendication 1, dans lequel le panier de récupération (22) est formé d'une paroi ayant des ouvertures s'étendant à travers la paroi.

6. Système de récupération selon la revendication 1, dans lequel le panier de récupération (22) comprend un élément tressé tubulaire.

7. Système de récupération selon la revendication 1, dans lequel le panier de récupération (22) est construit en un matériau comprenant un métal.

8. Système de récupération selon la revendication 1, dans lequel ladite attache (42) est raccordée au câble de commande, via une bague collectrice (50) positionnée sur le câble de commande (36) entre une paire d'éléments de retenue (52).

9. Système de récupération selon la revendication 1, comprenant en outre un système de verrouillage de câble (44) associé à l'élément pousseur (16) et ledit système de verrouillage de câble (44) verrouille le câble de commande (36) en place relativement à l'élément pousseur (16).
